# EUROPEAN PATENT APPLICATION

(11) **EP 3 967 708 A1**
(43) Date of publication of application: **16.03.2022**
(21) Application number: 20801746.7
(22) Date of filing: 30.04.2020
(51) Int. Cl.: C07K 16/24, C12N 15/13, C12N 1/21, C12N 15/62, A61P 35/00, A61P 37/04, A61K 39/395

(54) **ANTI-TNF-alpha HUMANIZED MONOCLONAL ANTIBODY TCX060 HAVING LOW IMMUNOGENICITY AND LOW ADCC/CDC FUNCTION, AND USE THEREOF**

(30) Priority: 07.05.2019 CN 201910376223
(71) Applicant: Abmax Biopharmaceuticals, Beijing 101111 (CN)
(72) Inventor: SUN, Le, Beijing 101111 (CN); REN, Wenlin, Beijing 101111 (CN)
(74) Representative: Micheli & Cie SA
(86) International application number: PCT/CN2020/088253
(87) International publication number: WO 2020/224529

(57) **Abstract**

Provided is an anti-TNF-α humanized monoclonal antibody, TCX060, wherein the antibody is obtained by means of modifying Certolizumab as follows: (1) removing PEG modification; (2) transforming same into a full-length antibody of a human IgG1 subtype; and (3) inserting a flexible amino acid fragment between CDR3 and CH2 regions.

## Description

### Cross-reference to related application

The present application claims priority to Chinese Patent Application No. 201910376223.0, filed on May 7, 2019, the entire disclosure of which is incorporated herein by reference in its entirety.

### Technical Field

The present invention relates to the field of biotechnology, in particular to an anti-TNF-a humanized monoclonal antibody TCX060 having low immunogenicity and low ADCC/CDC function and use thereof.

### Background Art

Tumor necrosis factor α (TNF-α) is a cytokine secreted by immune cells, and naturally secreted in inflammation and immune response. Studies have shown that the content of TNF-α has a tendency of up-regulation (for example, in the synovial fluid of the patients with rheumatoid arthritis (RA), the level of TNF-α is elevated) in patients with various chronic diseases (such as Crohn's disease, multiple sclerosis, rheumatoid arthritis, ulcerative colitis and the like), and it plays an important role in the process of pathological inflammation, joint destruction and the like. Human TNF-α has a molecular weight of 17 kDa and can exist *in vivo* as a monomer or trimer, and its active form is a trimer. TNF-α exerts its biological active functions by interacting with cell surface receptors p55 and p75. Currently, monoclonal IgG antibodies or soluble TNF-α receptors are generally used to neutralize TNF-α in the body.

At present, the commercially available monoclonal antibodies targeting TNF-α mainly include Etanercept, Infliximab, Adalimumab, Certolizumab and the like, wherein Etanercept is a fusion protein with two human TNF-α receptors (p75) coupled to the Fc end; Infliximab is a humanized anti-TNF-a murine monoclonal antibody, and Adalimumab is a humanized anti-TNF-a antibody. Radiolabeled TNF-α was used for the determination of binding capability and the results showed that Infliximab is capable of binding to both monomeric (inactive) and trimeric (active) types of soluble TNF-α; etanercept preferred more to bind to TNF-α and TNF-β in the form of active polymers, while Adalimumab only bound to TNF-α, rather than TNF-β.

Certolizumab, developed by Celltech UCB company, is a humanized monoclonal antibody that can specifically bind to TNF-α and block its interaction with cell surface TNF receptors p55 and p75. However, Certolizumab has strong immunogenicity in the host, and the percentage of patents who will develop immune response when using this monoclonal antibody is as high as 23% or more. The immune response may cause clearance of immune complex-mediated antibodies or fragments from the circulation and lead to repeated dosing which is inapplicable to therapy, thereby reducing therapeutic efficacy in patients and restricting the re-administrating of antibodies. Meanwhile, the primary drug Certolizumab needs to be used together with immunosuppressive agents, so that the risk of side effects is greatly increased. However, anti-TNF-a antibodies with low immunogenicity have not been reported yet. Therefore, there is an urgent need to develop antibodies against TNF-α with high affinity and specificity and low ADCC/CDC function and low immunogenicity.

### Summary of the Invention

In order to solve the technical problems in the prior art, the object of the present invention is to provide an anti-TNF-a humanized monoclonal antibody with low polymer content, low immunogenicity and low ADCC/CDC function, and the preparation method and use thereof.

In order to achieve the above-mentioned object, the technical solution of the present invention is as follows: in the present invention, the amino acid sequence of Certolizumab is analyzed using the commercial DNAStar^{™} software, and the results show that the immunogenicity of the original amino acid sequence of Certolizumab which is not modified with PEG is very low, however, the PEGylated Certolizumab may lead to the generation of polymers, which in turn leads to increased immunogenicity, that is, although PEGylation reduces the ADCC/CDC function, it leads to the problem of immununogenicity. On the basis of the above analysis, in the present invention, the monoclonal antibody sequence is modified on the basis of the sequence of Certolizumab, so as to maintain the high TNF-α binding affinity and specificity of Certolizumab, while reducing the generation of polymers and immunogenicity as well as ADCC/CDC function, and the specific modifications include:

First, in order to reduce the polymers and immunogenicity of Certolizumab, the PEG modification of Certolizumab is removed, however, the ADCC/CDC function of the antibody with the PEG modification removed is increased;

Further, based on the mechanism of action of inducing ADCC/CDC by the antibody, the resultant Certolizuma is transformed into a human IgG1 full-length antibody. On this basis, the antibody structure is analyzed using a protein structure analysis software such as Pymol and the like, and a relatively flexible region was found between the variable region and the constant region of the antibody, and then modified with insertion of flexible amino acids; finally, by inserting a flexible amino acid fragment between the CDR3 region and the CH2 region of the heavy chain of the antibody, mechanical stress transmission generated after the variable region of the antibody binds to the antigen is cut off, so that the binding site of the constant region of the heavy chain of the antibody for the Fc receptor and/or complement cannot be fully exposed, thereby weakening the binding of the antibody to killer cells that express IgG Fc receptors, such as NK cells, macrophages, neutrophils and the like or the binding to complements, and further ensuring that the antibody cannot induce or reduce the signals that induce ADCC and CDC.

A first object of the present invention is to provide an anti-TNF-a humanized monoclonal antibody TCX060, which is obtained by means of modifying Certolizumab as follows:
(1) removing PEG modification;
(2) transforming the resultant antibody into a full-length antibody of a human IgG1 subtype by adding a heavy chain constant region of a modified human IgG1 subtype antibody;
(3) inserting a flexible amino acid fragment between CDR3 and CH2 regions of the heavy chain of the obtained full-length antibody of a human IgG1 subtype;
   the full-length sequence for the light chain and the variable region sequence for the heavy chain of Certolizumab are shown in SEQ ID NO. 1 and SEQ ID NO. 2, respectively;
   the anti-TNF-a humanized monoclonal antibody TCX060 has the functions of binding to human TNF-α and blocking the binding of human TNF-α to TNF receptor.

In the above-mentioned step (3), inserting a flexible amino acid fragment between CDR3 and CH2 regions of the obtained full-length antibody of a human IgG1 subtype can block the stress transmission between the variable region and the constant region of the antibody, so that the binding site of the antibody to the Fc receptor and/or complement cannot be fully exposed, thereby effectively reducing the ADCC/CDC effect of the antibody.

In order to better block the mechanical stress transmission generated after the antibody variable region binds to the antigen, preferably, the insertion position of the flexible amino acid fragment is between amino acid residues at positions 237 and 238 of the sequence shown in SEQ ID NO.3. The flexible amino acid fragment contains one or more glycine or serine.

Further preferably, the sequence of the flexible amino acid fragment is GGGS, GGSGGS or GSGSGS.

As a preferred embodiment of the present invention, the insertion position of the flexible amino acid fragment is between amino acid residues at positions 237 and 238 of the sequence shown in SEQ ID NO.3; and the sequence of the flexible amino acid fragment is GGGS.

In the present invention, a large number of heavy chains of the anti-TNF-a humanized monoclonal antibody obtained based on the above-mentioned modification are screened, and the heavy chain of the anti-TNF-a humanized monoclonal antibody with the optimal performance in the aspects of immunogenicity and ADCC/CDC function is obtained, the full-length sequence of which is shown in SEQ ID NO.4 or is an amino acid sequence of a polypeptide having the same functions obtained by substitution, deletion or insertion of one or more amino acids in the amino acid sequence shown in SEQ ID NO.4.

In the present invention, the above-mentioned "amino acid sequence of a polypeptide having the same functions obtained by substitution, deletion or insertion of one or more amino acids" refers to a sequence being different from the specified sequence at one or more amino acid residues but retaining the biological activity of the obtained molecule, which may be a "conservatively modified variant" having the full-length heavy chain of the amino acid sequence shown in SEQ ID NO.4 or obtained by modification of "conservative amino acid substitution", "conservatively modified variants" or "conservative amino acid substitutions" refers to amino acid substitutions known to a person skilled in the art, and such substitutions are generally made without altering the biological activity of the resulting molecule. In general, it is recognized by a person skilled in that art that a single amino acid substitution in a non-essential region of a polypeptide does not substantially alter the biological activity. Exemplary substitutions are preferably carried out according to those substitutions shown in table 1:

**Table 1. Exemplary conservative amino acid substitutions table**

| Original residue | Conservative substitution |
|---|---|
| Ala (A) | Gly, Ser |
| Arg (R) | Lys, His |
| Asn (N) | Gln, His |
| Asp (D) | Glu, Asn |
| Cys (C) | Ser, Ala |
| Gln (Q) | Asn |
| Glu (E) | Asp, Gln |
| Gly (G) | Ala |
| His (H) | Asn, Gln |
| Ile (I) | Leu, Val |
| Lys (K) | Arg, His |
| Met (M) | Leu, Ile, Tyr |
| Phe (F) | Tyr, Met, Leu |
| Pro (P) | Ala |
| Ser (S) | Thr |
| Thr (T) | Ser |
| Trp (W) | Tyr, Phe |
| Tyr (Y) | Trp, Phe |
| Val (V) | Ile, Leu |

The heavy chain of the above-mentioned sequence as shown in SEQ ID NO.4 can be combined with the light chain of a modified or unmodified Certolizumab to effectively reduce the ADCC/CDC function of the non-PEGylated Certolizumab while having low immunogenicity.

By screening the monoclonal antibody with the above-mentioned light chain and heavy chain full-length sequences, the present invention obtains an optimal human TNF-α monoclonal antibody TCX060 composed of a combination of the full-length light chain and the full-length heavy chain, wherein the heavy chain full-length amino acid sequence is shown in SEQ ID NO.4, the light chain full-length amino acid sequence is shown in SEQ ID NO.1, the monoclonal antibody retains the antigen binding site of Certolizumab antibody and has similar expression level with Certolizumab antibody, and similar binding affinity to human TNF-α with Certolizumab antibody, and can block the binding of TNF-α to cell surface TNF receptors p55 and p75 specifically; its ADCC/CDC function is similar to that of the PEGylated Certolizumab antibody, but the monoclonal antibody has lower immunogenicity compared with the Certolizumab antibody.

A second object of the present invention is to provide a gene encoding the anti-TNF-a humanized monoclonal antibody TCX060.

The gene encoding the anti-TNF-a humanized monoclonal antibody TCX060 includes any nucleic acids encoding a heavy chain or light chain of the anti-TNF-a humanized monoclonal antibody provided by the present invention; according to the degeneracy of codons, it may be nucleic acids encoding the full-length amino acid sequence of the light chain and heavy chain obtained by codon optimization according to any host codon preference.

Preferably, in the above-mentioned gene, the nucleotide sequence encoding the full-length heavy chain is shown in SEQ ID NO.5, and the nucleotide sequence encoding the full-length light chain is shown in SEQ ID NO.6.

A third object of the present invention is to provide a biomaterial comprising the gene encoding the anti-TNF-a humanized monoclonal antibody TCX060.

The biological material comprises an expression cassette, a vector, a host cell, an engineering bacterium or a cell line.

A fourth object of the present invention is to provide a method for preparing the anti-TNF-a humanized monoclonal antibody TCX060, which is achieved by expressing the genes encoding the full-length heavy chain and the full-length light chain.

As one embodiment of the present invention, the method for preparing the anti-TNF-a humanized monoclonal antibody TCX060 comprises the following steps:
(1) synthesizing the full-length heavy chain gene and the full-length light chain gene of the anti-TNF-a humanized monoclonal antibody;
(2) ligating the above-mentioned synthetic genes into an expression vector;
(3) transforming the expression vector ligated with the above-mentioned full-length heavy chain gene and the full-length light chain gene of the anti-TNF-a humanized monoclonal antibody into a host cell;
(4) screening for a host cell stably expressing the anti-TNF-a humanized monoclonal antibody, culturing the host cell, and expressing the anti-TNF-a humanized monoclonal antibody;
(5) extracting and purifying the anti-TNF-a humanized monoclonal antibody.

A fifth object of the present invention is to provide the use of the anti-TNF-a humanized monoclonal antibody TCX060 or the gene encoding the anti-TNF-a humanized monoclonal antibody TCX060 or a biological material comprising the gene in the preparation of a medicament targeting human TNF-α.

Preferably, the medicament targeting human TNF-α is a medicament for preventing or treating tumor, inflammation or autoimmune disease.

The diseases include but are not limited to Crohn's disease, rheumatoid arthritis, psoriatic arthritis, active ankylosing spondylitis and the like.

A sixth object of the present invention is to provide the use of the anti-TNF-a humanized monoclonal antibody TCX060 or the gene encoding the anti-TNF-a humanized monoclonal antibody TCX060 or the biological material comprising the gene in the preparation of a human TNF-α detection reagent.

A seventh object of the present invention is to provide a medicament or a detection reagent comprising the anti-TNF-a humanized monoclonal antibody TCX060.

The medicament comprising the anti-TNF-a humanized monoclonal antibody TCX060 may also comprise other active ingredients or excipients permitted in the pharmaceutical field.

The beneficial effects of the present invention are as follows:
In the present invention, an anti-TNF-a humanized monoclonal antibody is obtained through artificial modification on the basis of Certolizumab, the humanized anti-TNF-a monoclonal antibody provided by the present invention has the same antigen binding site for TNF-α as Certolizumab, but its antibody conformation, immunogenicity and other properties are different from those of Certolizumab. The humanized anti-TNF-a monoclonal antibody TCX060 provided by the present invention retains the antigen affinity and specificity of Certolizumab, and its ADCC/CDC induction function is similar to that of the PEGylated Certolizumab, but its immunogenicity is significantly lower than that of Certolizumab; meanwhile, the production process of the humanized anti-TNF-a monoclonal antibody provided by the present invention is more simplified. In one aspect, lower immunogenicity will reduce the risk of side effect of the drug caused by immunological reactions resulted from the immunogenicity of antibodies in human bodies, and meanwhile, the reduction of immunogenicity can prolong the *in vivo* half-life of the antibody drug prepared by the humanized anti-TNF-a monoclonal antibody provided by the present invention; and avoid the reduction of drug effect caused by ADA of the antibody drug to a greater extent; in another aspect, it can reduce the dosage of the antibody drug to reduce the cost of treatment; and in yet another aspect, it can get rid of the mode of combined administration of the anti-TNF-a monoclonal antibody drug and the immunosuppressive agent, and achieve the effect of single administration of the anti-TNF-a monoclonal antibody drug, remove the side effects brought by the combination with the immunosuppressive agent, and improve the medication safety. The humanized anti-TNF-a monoclonal antibody TCX060 provided by the present invention has great application potential and value and is expected to become an ideal biological targeted therapeutic antibody.

### Brief Description of the Drawings

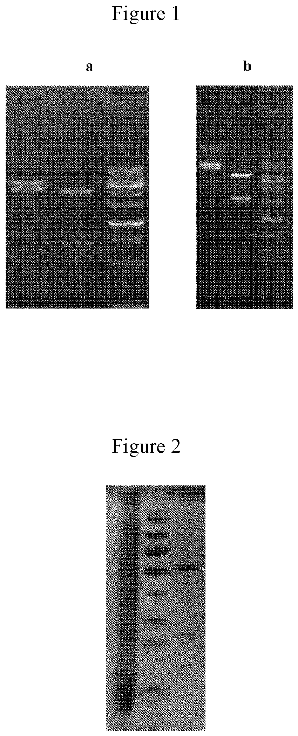

Figure 1 is a diagram showing the electrophoresis results of double digests identification of the expression vectors for Certolizumab coding gene in Example 2 of the present invention and for H2L0 monoclonal antibody coding gene provided by the present invention, in which "a" is the result of double digests by Hind III and EcoRI of the plasmid for light chain L0 of Certolizumab, and the lanes from left to right are: the plasmid before digestion, the plasmid after digestion and the DNA marker, respectively; and "b" is the result of double digests by Hind III and EcoRI of the plasmid for heavy chain H2 of the monoclonal antibody TCX060 provided by the present invention, and the lanes from left to right are: the plasmid before digestion, the plasmid after digestion and the DNA marker, respectively.
Figure 2 is an SDS-PAGE electrophoresis diagram of the monoclonal antibody TCX060 in Example 4 of the present invention, and the lanes from left to right are: expression supernatant, Marker, and purified antibody, respectively.

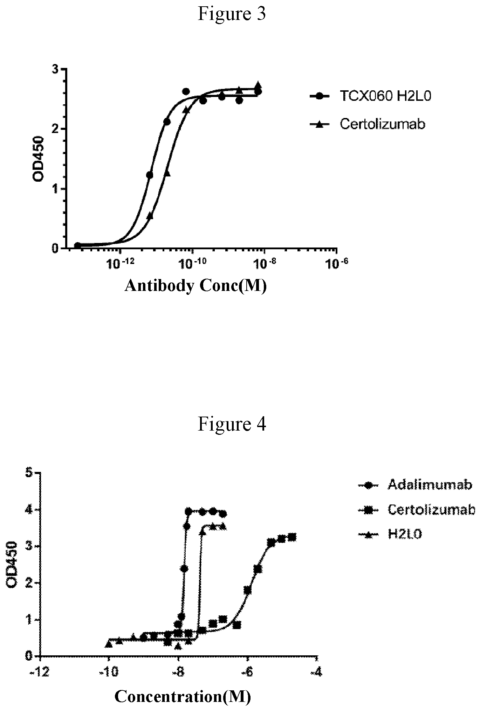

Figure 3 shows the affinity EC₅₀ of the monoclonal antibody TCX060 in Example 5 of the present invention; wherein H2L0 represents TCX060.
Figure 4 shows the results of the TNF-α-mediated cell killing experiment of the monoclonal antibody TCX060 in Example 5 of the present invention; wherein Adalimumab represents the primary drug of Adalimumab, Certolizumab represents the primary drug of Certolizumab, and H2L0 represents the antibody TCX060 H2L0 of the present invention.

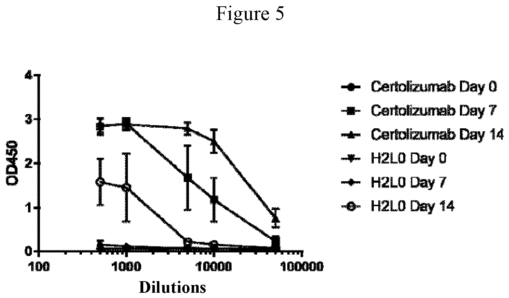

Figure 5 shows the mouse ADA evaluation result of the monoclonal antibody TCX060 in Example 6 of the present invention; wherein Certolizumab Day 0, Certolizumab Day 7, and Certolizumab Day 14 represent the serum samples before immunization, on the 7th day after the immunization by the primary drug of Certolizumab, and on the 14th day after the immunization by the primary drug of Certolizumab, respectively, and H2L0 Day 0, H2L0 Day 7, H2L0 Day 14 represent the serum samples before immunization, and on the 7th day after the immunization by the antibody drug of TCX060 H2L0 of the present invention, and on the 14th day after the immunization by the antibody drug of TCX060 H2L0 of the present invention, respectively.

### Specific Modes for Carrying Out the Embodiments

The preferred embodiments of the present invention will be described in detail below in conjunction with Examples. It should be understood that the following Examples are given for illustrative purposes only, and are not intended to limit the scope of the present invention. A person skilled in the art can make various modifications and substitutions to the present invention without departing from the purpose and spirit of the present invention.

Unless otherwise specified, the experimental methods used in the following Examples are all conventional methods.

Unless otherwise specified, the materials, reagents and the like used in the following Examples are commercially available.

### Example 1: Analysis and design of the Certolizumab monoclonal antibody with low ADCC/CDC function and reduced immunogenicity

In the present invention, the original sequence of Certolizumab was analyzed and evaluated using commercial DNAStar^{™} software, and the analysis result shows that the immunogenicity of the original Certolizumab sequence is very low. It is speculated that after the original Certolizumab is subjected to PEGylation so as to remove ADCC/CDC function, the polymers of the antibody increases, which in turn lead to up to 23% ADA of the drug. Therefore, it is necessary to remove the PEGylation modification of the original Certolizumab while reducing its ADCC/CDC function. The amino acid sequences of the full-length light chain and the variable region of the heavy chain of the original Certolizumab are shown in SEQ ID NO. 1 and SEQ ID NO. 2, respectively.

Firstly, the original Certolizumab was subjected to the removal of PEG modification, and subjected to modification to give the human IgG1 subtype complete antibody. On this basis, by looking for a relatively flexible region between the variable region and the constant region of the human IgG1 subtype complete antibody obtained by the modification, the mechanical stress transmission generated after the antibody variable region binds to an antigen was cut off by inserting a flexible amino acid sequence in the flexible region, so that the binding site of the constant region of the antibody heavy chain for the Fc receptor and/or complement cannot be fully exposed, the binding of the antibody to killer cells that express IgG Fc receptors such as NK cells, Macrophages and neutrophils and the like or binding to complement is reduced, and thereby the antibody cannot induce ADCC or CDC or has reduced signals that induce ADCC and CDC. Through the analysis by Pymol software, it is determined that inserting a flexible amino acid sequence between the heavy chain CDR3 and CH2 regions of the above-mentioned human IgG1 subtype complete antibody of Certolizumab can better block the stress transmission between the variable region and the constant region of the antibody after the antibody binds to the antigen, and will not significantly increase the formation of antibody polymers while reducing the ADCC/CDC function. Through a large number of sequence analysis and structural simulation predictions, the human IgG1 subtype complete antibody of Certolizumab the heavy chain sequence of which is shown in SEQ ID NO. 3 is identified, and it is determined that the heavy chain H2 of the modified human IgG1 subtype complete antibody, the sequence of which is shown in SEQ ID NO. 4, was obtained by inserting the flexible amino acid sequence GGGS between the amino acid residues at positions 237 and 238 of the sequence shown in SEQ ID NO. 3; through preliminary evaluation of the comprehensive performance of the monoclonal antibodies obtained by combination of different light and heavy chains, an anti-TNF-a humanized monoclonal antibody H2L0 consisting of a light chain L0 (the original light chain sequence of Certolizumab, SEQ ID NO. 1) and a heavy chain H2 (SEQ ID NO. 4) was finally obtained. The monoclonal antibody was designated TCX060.

### Example 2: Construction of an expression vector for anti-TNF-α humanized monoclonal antibody TCX060

According to the amino acid sequences of the full-length heavy chain and light chain of the anti-TNF-a fully humanized monoclonal antibody H2L0 obtained in Example 1 and the codon preference of the host, the nucleotide sequences encoding the heavy chain H2 and the light chain L0 were designed (wherein the nucleotide sequence of L0 is shown in SEQ ID NO. 6, and the nucleotide sequence of H2 is shown in SEQ ID NO. 5), the restriction sites on both ends of the light chain sequence are designed as Hind III + EcoR I, the restriction sites on both ends of the heavy chain sequence are designed as Hind III + EcoR I, and the full-length heavy and light chain sequences carrying restriction sites were sent to Genewiz, Inc. to synthesize the whole gene sequence, the ligation vector used for synthesis was pUC57. As expression vectors, pEE12.4 (for the expression of heavy chain) and pEE6.4 (for the expression of light chain) were used, the above expression vectors and the synthesized gene sequences were subjected to corresponding double digests, respectively, and the target gene and the expression vector obtained by enzyme digestion were separated by agarose gel electrophoresis, subsequently, the target band was cut off and recovered by Qiagen Gel Extraction Kit, the resultant was subjected to ligation according to the T4 DNA enzyme ligation system at 16°C overnight, then the ligation product was transformed into *Escherichia. coli* DH5α, and the obtained transformants were subjected to PCR identification, plasmid extraction and sequencing to obtain expression vectors carrying the genes of the full-length heavy chain H2 and the full-length light chain L0, respectively. The original Certolizumab was used as control, the pEE12.4 expression vector carrying the heavy chain H0 of the original Certolizumab (the sequence is shown in SEQ ID NO. 2) was constructed, and the construction method was the same as that of the heavy chain H2 expression vector.

The electrophoresis results of double digests identification of the expression vector carrying the above-mentioned full-length heavy and light chain genes are shown in FIG. 1.

### Example 3: Transient expression of anti-TNF-α humanized monoclonal antibody TCX060

The *E. coli* DH5α strains obtained in Example 2 carrying the expression vectors of the genes of the full-length heavy chains H2, H0 and light chain L0, respectively, were cultured, the culture was harvested, and the expression vectors carrying the gene of the full-length heavy and light chains were extracted and purified with Qiagen UltraPure Plasmid DNA Purification Kit. The above-mentioned purified plasmid DNA was transformed into 293F cells using Invitrogen's liposome method kit, and for the transformation method, please refer to the kit instructions.

The positive cells obtained by transformation were subjected to antibody expression, and the expression level of the monoclonal antibody TCX060 with the combination H2L0 of the heavy and light chains is shown in Table 2.

**Table 2. Expression levels of human antibodies (mg/L)**

| Monoclonal antibody | Expression level µg/mL (mg/L) |
|---|---|
| TCX060 | 6 |

The antibody H2L0 obtained by the above expression was subjected to an experiment using pre-coating of TNF-α, the monoclonal antibody H2L0 was added into a 96-well plate pre-coated with TNF-α, and the activity of the secreted antibody binding TNF-α was preliminarily evaluated by indirect ELISA method. The test results are shown in Table 3. NC is negative control using diluent for antibody.

**Table 3. Evaluation results of activity of monoclonal antibody TCX060**

| | Original Certolizumab | H2L0 |
|---|---|---|
| ECso(M) | 9.374e-011 | 2.464e-011 |

The results in Table 3 show that the monoclonal antibody TCX060 corresponding to the combination of H2L0 was expressed, and the results in Table 3 show that the monoclonal antibody TCX060 can recognize TNF-α.

### Example 4: Expression and purification of the anti-TNF-α humanized monoclonal antibody TCX060

According to the detection results in Example 3, the expression vector for the monoclonal antibody TCX060 of the combination H2L0 of heavy and light chains was subjected to stable transformation.

The 293F cells was transformed by transient transformation method, and the cell culture supernatant was collected on day 7 after transformation using 293 mudium and transformation reagents from Zhuhai Kairui company.

The culture supernatant of the monoclonal antibody TCX060 (H2L0 combination) and the original Certolizumab (H0L0) was directly separated, and purified by MabSelect Sure LX from GE company. For details of the experimental process, please refer to instructions of UCB company, and the purified product was quantitatively detected using a UV spectrophotometer, the calculation formula is as follows:
Concentration calculation: After reading the OD₂₈₀ of the collected elution peaks, calculate the concentration. Antibody concentration = OD₂₈₀/1.4.

After antibody purification, SDS-PAGE electrophoresis was performed for verification. The results are shown in Figure 2, the electrophoresis results show that the purified antibody has a purity of more than 80%, a relatively single band and an equivalent concentration, and can be used for later experiments.

### Example 5: Determination of biological activities of anti-TNF-α humanized monoclonal antibody TCX060

### 1. Evaluation of affinity:

In the present Example, the antibody EC₅₀ was measured by indirect ELISA to evaluate the antibody affinity.

The experimental method was as follows: TNF-α (purchased from Jin'an Technology Co., Ltd.) antigen was diluted with PBS to 1µg/mL. The diluted antigen was added to a 96-well plate at 100 µl/well, the 96-well plate was covered, and stored at 4°C overnight. The liquid in the well was shook off, and the 96-well plate was washed three times with PBS at 200 µl/well, and patted to dry. The 96-well plate was blocked with 5% milk-PBS at 200 µl/well for 1h, and patted every 15min. The liquid in the well was shook off, the 96-well plate was washed once with PBS at 200 µl/ well, and patted to dry. The purified antibodies (0 to 10µg/mL) was added with a gradient, and the names of antibodies are shown in Table 4. The antibody was diluted with 5% milk -PBS at 100 µl/well, incubated for 1h, and patted every 15min The liquid in the well was shook off, and the plate was washed three times with PBS at 200 µl/ well, and patted to dry. Secondary antibody diluted with 5% milk-PBS was added at 100 µl/well, incubated for 1h, and patted every 15min. TMB substrate was preheated at room temperature, the microplate reader was turned on for preheating. The 96-well plate was washed with PBS for 5 times at 250 µl/well, wherein the first three times were performed for 5min and the last two times were performed for 10min, and then patted to dry. TMB substrates A and B were added at 50 µl/well respectively for color developing at room temperature for 20min. A scanner was used to scan and record the pictures, stop solution was added at 50 µl/well, and OD₄₅₀ was readed with the microplate reader. The experimental data are shown in Table 4 and Figure 3.

**Table 4. Test results of EC₅₀ of monoclonal antibody TCX060**

| EC₅₀ (ng/mL) | | EC₅₀ (M) | |
|---|---|---|---|
| Certolizumab | H2L0 | Certolizumab | H2L0 |
| 39.81 | 19.95 | 2.65E-10 | 1.33E-10 |

The above results indicate that the affinity of the monoclonal antibody TCX060 to human TNF-α was slightly higher than that of the original Certolizumab.

### 2. Cytotoxic effect experiment

The CCK-8 kit was used to detect the apoptosis of mouse fibroblast cell line L929. The specific steps were as follows:
100 µl of three-fold serially-diluted monoclonal antibody (diluted by RPMI-1640 medium containing 10% FBS) (the concentration of monoclonal antibody TCX060 added is set up and down 3 to 5 gradient concentrations according to the IC₅₀ of the experiment for the Certolizumab assay mentioned by FDA, respectively) was added into a 96-well plate, and then 50 µl of rhTNF-α (diluted by RPMI-1640 medium containing 10% FBS) with a final concentration of 500pg/ml was added, and the resultant was incubated for 30 minutes at room temperature. 50 µl of L929 cells (containing a final concentration of 1µg/ml of actinomycin-D) were added at 5×10⁴/well. The resultant was incubated overnight in a 37°C incubator (18 to 24h). Controls include a negative control and a positive control. In the negative control, only RPMI-1640 and cells were added, while in the positive control, only rhTNFα and cells were added, the concentration of rhTNFα varied in a gradient from 2 ng/ml to 8.2 pg/ml. 20 µL of CCK8 solution was added to each well (be careful not to generate bubbles in the well, so as not to affect the reading of OD values). The culture plate was incubated in an incubator at 37°C for 1 to 4h. The absorbance at 450 nm was measured with a microplate reader. If the OD value is not determined for the moment, 10 µL of 0.1M HCL solution or 1% w/v SDS solution can be added to each well, and the plate can be covered and stored at room temperature to avoid light. The absorbance is measured within 24 hours so that it will not change.

The cell killing rate and IC50 of the antibody at different concentrations were calculated.

Calculation formula: (Monoclonal antibody TCX060 treated cells - TNF-α treated cells)/TNF-α treated cells × 100%.

The experimental results are shown in Fig.4. The IC50 data are shown in Table 5, and the IC50 of TCX060 (H2L0) is comparable to that of the original Adalimumab, and the IC50 of TCX060 (H2L0) is lower than that of Certolizumab.

**Table 5. IC50 test results of monoclonal antibody TCX060**

| | TA001 (the original Adalimumab) | Q002 (Certolizumab) | TCX060 H2L0 |
|---|---|---|---|
| IC50 (M) | 1.478e-008 | 1.305e-006 | ∼ 4.249e-008 |

### Example 6: Immunogenicity evaluation of anti-TNF-α humanized monoclonal antibody TCX060

The immunization experiment of anti-TNF-a humanized monoclonal antibody TCX060 was carried out in mice, and the immunogenicity of TCX060 was analyzed. The details are as follows:
1) basic immunization: TCX060 (H2L0) and the original Certolizumab was mixed with Freund's complete adjuvant in equal volume and fully emulsified, and injected subcutaneously at different points, the injection amount for each Balb/c mouse was 70 µg; and
2) booster immunization: an emulsion of antigen and Freund's incomplete adjuvant was used for booster immunization.

After the above experiment was completed, the ELISA detection test was performed according to the following experimental method: the TCX060 (H2L0) or the original Certolizumab was diluted with PBS to 1 µg/ml. The diluted antigen was added to a 96-well plate at 100 µl/well, the plate was covered and stored at 4°C overnight. The liquid in the well was shook off, and the 96-well plate was washed with PBS for three times at 200 µl/well, and patted to dry. The 96-well plate was blocked with 5% milk-PBS at 200 µl/well for 1h, patted every 15min. The liquid in the well was shook off and the plate was washed with PBS once at 200 µl/well, and patted to dry. Serum samples on different days after immunization with different dilution ratios (1:500/1:1000/1:5,000/1:10,000/1:50,000, diluted with 5% milk-PBS) were added at 100 µl/well, incubated for 1h, and patted every 15min. The liquid in the well was shook off, and the 96-well plate was washed three times with PBS at 200 µl/well, and patted to dry. Secondary antibody diluted with 5% milk-PBS was added at 100 µl/well, incubated for 1h, and patted every 15min. TMB substrate was preheated at room temperature, the microplate reader was turned on for preheating. The 96-well plate was washed with PBS for 5 times at 250 µl/well, wherein the first three times were performed for 5min, and the last two times were performed for 10min, and patted to dry. TMB substrates A and B were added at 50 µl/well respectively for color developing at room temperature for 20min. A scanner was used to scan and record the pictures, stop solution was added at 50 µl/well, and OD₄₅₀ was readed with a microplate reader.

The results are shown in Figure 5. The results show that the anti-TNF-α humanized monoclonal antibody TCX060 H2L0 of the present invention has a significant reduction in serum antibody titer compared with Certolizumab, and therefore, the immunogenicity of H2L0 has been effectively reduced compared with Certolizumab.

Although the general description and specific embodiments have been used to describe the present invention in detail above, it is obvious to a person skilled in the art that some modifications or improvements can be made on the basis of the present invention. Therefore, all these modifications or improvements made without departing from the spirit of the present invention belong to the scope of the present invention.

### Industrial applicability

The present invention provides an anti-TNF-α humanized monoclonal antibody TCX060 having low immunogenicity and low ADCC/CDC function and use thereof. The anti-TNF-α humanized monoclonal antibody TCX060 provided by the present invention is obtained by modifying Certolizumab as follows: (1) removing PEG modification; (2) modifying Certolizumab into a full-length antibody of a human IgG1 subtype; and (3) inserting a flexible amino acid fragment between CDR3 and CH2 regions. TCX060 has a binding affinity to human TNF-α similar to that of Certolizumab, can specifically block the binding of TNF-α to the cell surface TNF receptor, has a ADCC/CDC function similar to that of PEGylated Certolizumab, but the immunogenicity is significantly lower than that of Certolizumab, which can reduce the risk of generating immunogenicity of the antibody *in vivo* while removing the ADCC/CDC function. The antibody TCX060 has good economic value and application prospect.

## Claims

1. An anti-TNF-a humanized monoclonal antibody TCX060, wherein, the monoclonal antibody TCX060 is obtained by modifying Certolizumab as follows:
(1) removing PEG modification;
(2) transforming the resultant into a full-length antibody of a human IgG1 subtype by adding a heavy chain constant region of a modified human IgG1 subtype antibody; and
(3) inserting a flexible amino acid fragment between CDR3 and CH2 regions of the heavy chain of the obtained full-length antibody of a human IgG1 subtype;
the full-length sequence of the light chain and the sequence of the variable region of the heavy chain of Certolizumab are shown in SEQ ID NO. 1 and SEQ ID NO. 2, respectively;
the anti-TNF-α humanized monoclonal antibody TCX060 has the functions of binding to human TNF-α and blocking the binding of human TNF-α to TNF receptor.

2. The monoclonal antibody TCX060 according to claim 1, wherein, the insertion position of the flexible amino acid fragment is between amino acid residues at positions 237 and 238 of the sequence shown in SEQ ID NO.3; and the flexible amino acid fragment contains one or more glycine and serine.

3. The monoclonal antibody TCX060 according to claim 2, wherein, the sequence of the flexible amino acid fragment is one selected from GGGS, GGSGGS and GSGSGS.

4. The monoclonal antibody TCX060 according to any one of claims 1 to 3, wherein, the full-length sequence of the heavy chain of the monoclonal antibody TCX060 is shown in SEQ ID NO.4 or is an amino acid sequence of a polypeptide having the same functions obtained by substitution, deletion or insertion of one or more amino acids in the amino acid sequence shown in SEQ ID NO.4.

5. A gene encoding the monoclonal antibody TCX060 according to anyone of claims 1 to 4.

6. The gene according to claim 5, wherein, the nucleotide sequence encoding the full-length heavy chain is shown in SEQ ID NO. 5, and the nucleotide sequence encoding the full-length light chain is shown in SEQ ID NO. 6.

7. A biological material comprising the gene according to claim 5 or 6, wherein, the biological material comprises an expression cassette, a vector, a host cell, an engineering bacteria or a cell line.

8. Use of the monoclonal antibody TCX060 according to any one of claims 1 to 4 or the gene according to claim 5 or 6 or the biological material according to claim 7 in the preparation of a medicament targeting human TNF-α;
preferably, the medicament targeting human TNF-α is a medicament for preventing or treating tumor, inflammation or autoimmune disease.

9. Use of the monoclonal antibody TCX060 according to any one of claims 1 to 4 or the gene according to claim 5 or 6 or the biological material according to claim 7 in the preparation of a human TNF-α detection reagent.

10. A medicament or detection reagent comprising the monoclonal antibody TCX060 according to any one of claims 1 to 4.


sequence listing

<110>  ABMAX BIOPHARMACEUTICALS Co., Ltd.

<120>  Anti-TNF-α humanized monoclonal antibody TCX060 having low
immunogenicity and low ADCC/CDC function, and use thereof

<130>  KHP213110188.5

<160>  6

<170>  SIPO Sequence Listing 1.0

<210>  1
<211>  214
<212>  PRT
<213>  Artificial Sequence

<220>
<223>  Laboratory synthesis

<400>  1
Asp Ile Gln Met Thr Gln Ser Pro Ser Ser Leu Ser Ala Ser Val Gly
1               5                   10
15
Asp Arg Val Thr Ile Thr Cys Lys Ala Ser Gln Asn Val Gly Thr Asn
            20                  25
30
Val Ala Trp Tyr Gln Gln Lys Pro Gly Lys Ala Pro Lys Ala Leu Ile
        35                  40                  45
Tyr Ser Ala Ser Phe Leu Tyr Ser Gly Val Pro Tyr Arg Phe Ser Gly
    50                  55                  60
Ser Gly Ser Gly Thr Asp Phe Thr Leu Thr Ile Ser Ser Leu Gln Pro
65                  70                  75
80
Glu Asp Phe Ala Thr Tyr Tyr Cys Gln Gln Tyr Asn Ile Tyr Pro Leu
                85                  90
95
Thr Phe Gly Gln Gly Thr Lys Val Glu Ile Lys Arg Thr Val Ala Ala
            100                 105
110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155
160
Glu Ser Val Thr Glu Gln Asp Ser Lys Asp Ser Thr Tyr Ser Leu Ser
                165                 170
175
Ser Thr Leu Thr Leu Ser Lys Ala Asp Tyr Glu Lys His Lys Val Tyr
            180                 185
190
Ala Cys Glu Val Thr His Gln Gly Leu Ser Ser Pro Val Thr Lys Ser
        195                 200                 205
Phe Asn Arg Gly Glu Cys
    210

<210>  2
<211>  229
<212>  PRT

<213>  Artificial Sequence

<220>
<223>  Laboratory synthesis

<400>  2
Glu Val Gln Leu Val Glu Ser Gly Gly Gly Leu Val Gln Pro Gly Gly
1               5                   10
15
Ser Leu Arg Leu Ser Cys Ala Ala Ser Gly Tyr Val Phe Thr Asp Tyr
            20                  25
30
Gly Met Asn Trp Val Arg Gln Ala Pro Gly Lys Gly Leu Glu Trp Met
        35                  40                  45
Gly Trp Ile Asn Thr Tyr Ile Gly Glu Pro Ile Tyr Ala Asp Ser Val
    50                  55                  60
Lys Gly Arg Phe Thr Phe Ser Leu Asp Thr Ser Lys Ser Thr Ala Tyr
65                  70                  75
80
Leu Gln Met Asn Ser Leu Arg Ala Glu Asp Thr Ala Val Tyr Tyr Cys
                85                  90
95
Ala Arg Gly Tyr Arg Ser Tyr Ala Met Asp Tyr Trp Gly Gln Gly Thr
            100                 105
110
Leu Val Thr Val Ser Ser Ala Ser Thr Lys Gly Pro Ser Val Phe Pro
        115                 120                 125
Leu Ala Pro Ser Ser Lys Ser Thr Ser Gly Gly Thr Ala Ala Leu Gly
    130                 135                 140
Cys Leu Val Lys Asp Tyr Phe Pro Glu Pro Val Thr Val Ser Trp Asn
145                 150                 155
160
Ser Gly Ala Leu Thr Ser Gly Val His Thr Phe Pro Ala Val Leu Gln
                165                 170
175
Ser Ser Gly Leu Tyr Ser Leu Ser Ser Val Val Thr Val Pro Ser Ser
            180                 185
190
Ser Leu Gly Thr Gln Thr Tyr Ile Cys Asn Val Asn His Lys Pro Ser
        195                 200                 205
Asn Thr Lys Val Asp Lys Lys Val Glu Pro Lys Ser Cys Asp Lys Thr
    210                 215                 220
His Thr Cys Ala Ala
225

<210>  3
<211>  448
<212>  PRT
<213>  Artificial Sequence

<220>
<223>  Laboratory synthesis

<400>  3
Glu Val Gln Leu Val Glu Ser Gly Gly Gly Leu Val Gln Pro Gly Gly
1               5                   10
15
Ser Leu Arg Leu Ser Cys Ala Ala Ser Gly Tyr Val Phe Thr Asp Tyr
            20                  25
30
Gly Met Asn Trp Val Arg Gln Ala Pro Gly Lys Gly Leu Glu Trp Met
        35                  40                  45

Gly Trp Ile Asn Thr Tyr Ile Gly Glu Pro Ile Tyr Ala Asp Ser Val
    50                          55                          60
Lys Gly Arg Phe Thr Phe Ser Leu Asp Thr Ser Lys Ser Thr Ala Tyr
65                          70                          75
80
Leu Gln Met Asn Ser Leu Arg Ala Glu Asp Thr Ala Val Tyr Tyr Cys
                            85                          90
95
Ala Arg Gly Tyr Arg Ser Tyr Ala Met Asp Tyr Trp Gly Gln Gly Thr
                  100                         105
110
Leu Val Thr Val Ser Ser Ala Ser Thr Lys Gly Pro Ser Val Phe Pro
            115                         120                         125
Leu Ala Pro Ser Ser Lys Ser Thr Ser Gly Gly Thr Ala Ala Leu Gly
    130                         135                         140
Cys Leu Val Lys Asp Tyr Phe Pro Glu Pro Val Thr Val Ser Trp Asn
145                         150                         155
160
Ser Gly Ala Leu Thr Ser Gly Val His Thr Phe Pro Ala Val Leu Gln
                        165                         170
175
Ser Ser Gly Leu Tyr Ser Leu Ser Ser Val Val Thr Val Pro Ser Ser
                  180                         185
190
Ser Leu Gly Thr Gln Thr Tyr Ile Cys Asn Val Asn His Lys Pro Ser
            195                         200                         205
Asn Thr Lys Val Asp Lys Lys Val Glu Pro Lys Ser Cys Asp Lys Thr
      210                         215                         220
His Thr Cys Pro Pro Cys Pro Ala Pro Glu Leu Leu Gly Gly Pro Ser
225                         230                         235
240
Val Phe Leu Phe Pro Pro Lys Pro Lys Asp Thr Leu Met Ile Ser Arg
                          245                         250
255
Thr Pro Glu Val Thr Cys Val Val Val Asp Val Ser His Glu Asp Pro
                    260                         265
270
Glu Val Lys Phe Asn Trp Tyr Val Asp Gly Val Glu Val His Asn Ala
            275                         280                         285
Lys Thr Lys Pro Arg Glu Glu Gln Tyr Asn Ser Thr Tyr Arg Val Val
    290                         295                         300
Ser Val Leu Thr Val Leu His Gln Asp Trp Leu Asn Gly Lys Glu Tyr
305                         310                         315
320
Lys Cys Lys Val Ser Asn Lys Ala Leu Pro Ala Pro Ile Glu Lys Thr
                        325                         330
335
Ile Ser Lys Ala Lys Gly Gln Pro Arg Glu Pro Gln Val Tyr Thr Leu
                340                         345
350
Pro Pro Ser Arg Glu Glu Met Thr Lys Asn Gln Val Ser Leu Thr Cys
            355                         360                         365
Leu Val Lys Gly Phe Tyr Pro Ser Asp Ile Ala Val Glu Trp Glu Ser
    370                         375                         380
Asn Gly Gln Pro Glu Asn Asn Tyr Lys Thr Thr Pro Pro Val Leu Asp
385                         390                         395
400
Ser Asp Gly Ser Phe Phe Leu Tyr Ser Lys Leu Thr Val Asp Lys Ser
                        405                         410
415
Arg Trp Gln Gln Gly Asn Val Phe Ser Cys Ser Val Met His Glu Ala

                    420                             425
430
Leu His Asn His Tyr Thr Gln Lys Ser Leu Ser Leu Ser Pro Gly Lys
            435                             440                             445

<210>  4
<211>  452
<212>  PRT
<213>  Artificial Sequence

<220>
<223>  Laboratory synthesis

<400>  4
Glu Val Gln Leu Val Glu Ser Gly Gly Gly Leu Val Gln Pro Gly Gly
1                   5                               10
15
Ser Leu Arg Leu Ser Cys Ala Ala Ser Gly Tyr Val Phe Thr Asp Tyr
                    20                              25
30
Gly Met Asn Trp Val Arg Gln Ala Pro Gly Lys Gly Leu Glu Trp Met
             35                             40                              45
Gly Trp Ile Asn Thr Tyr Ile Gly Glu Pro Ile Tyr Ala Asp Ser Val
      50                            55                              60
Lys Gly Arg Phe Thr Phe Ser Leu Asp Thr Ser Lys Ser Thr Ala Tyr
65                            70                              75
80
Leu Gln Met Asn Ser Leu Arg Ala Glu Asp Thr Ala Val Tyr Tyr Cys
                            85                              90
95
Ala Arg Gly Tyr Arg Ser Tyr Ala Met Asp Tyr Trp Gly Gln Gly Thr
                   100                             105
110
Leu Val Thr Val Ser Ser Ala Ser Thr Lys Gly Pro Ser Val Phe Pro
            115                             120                             125
Leu Ala Pro Ser Ser Lys Ser Thr Ser Gly Gly Thr Ala Ala Leu Gly
      130                           135                             140
Cys Leu Val Lys Asp Tyr Phe Pro Glu Pro Val Thr Val Ser Trp Asn
145                           150                             155
160
Ser Gly Ala Leu Thr Ser Gly Val His Thr Phe Pro Ala Val Leu Gln
                            165                             170
175
Ser Ser Gly Leu Tyr Ser Leu Ser Ser Val Val Thr Val Pro Ser Ser
                   180                             185
190
Ser Leu Gly Thr Gln Thr Tyr Ile Cys Asn Val Asn His Lys Pro Ser
            195                             200                             205
Asn Thr Lys Val Asp Lys Lys Val Glu Pro Lys Ser Cys Asp Lys Thr
      210                           215                             220
His Thr Cys Pro Pro Cys Pro Ala Pro Glu Leu Leu Gly Gly Gly Gly
225                           230                             235
240
Ser Gly Pro Ser Val Phe Leu Phe Pro Pro Lys Pro Lys Asp Thr Leu
                            245                             250
255
Met Ile Ser Arg Thr Pro Glu Val Thr Cys Val Val Val Asp Val Ser
                   260                             265
270
His Glu Asp Pro Glu Val Lys Phe Asn Trp Tyr Val Asp Gly Val Glu
            275                             280                             285
Val His Asn Ala Lys Thr Lys Pro Arg Glu Glu Gln Tyr Asn Ser Thr

290                 295                 300
Tyr Arg Val Val Ser Val Leu Thr Val Leu His Gln Asp Trp Leu Asn
305                 310                 315
320
Gly Lys Glu Tyr Lys Cys Lys Val Ser Asn Lys Ala Leu Pro Ala Pro
                325                 330
335
Ile Glu Lys Thr Ile Ser Lys Ala Lys Gly Gln Pro Arg Glu Pro Gln
            340                 345                 350
Val Tyr Thr Leu Pro Pro Ser Arg Glu Glu Met Thr Lys Asn Gln Val
        355                 360                 365
Ser Leu Thr Cys Leu Val Lys Gly Phe Tyr Pro Ser Asp Ile Ala Val
    370                 375                 380
Glu Trp Glu Ser Asn Gly Gln Pro Glu Asn Asn Tyr Lys Thr Thr Pro
385                 390                 395
400
Pro Val Leu Asp Ser Asp Gly Ser Phe Phe Leu Tyr Ser Lys Leu Thr
                405                 410
415
Val Asp Lys Ser Arg Trp Gln Gln Gly Asn Val Phe Ser Cys Ser Val
            420                 425
430
Met His Glu Ala Leu His Asn His Tyr Thr Gln Lys Ser Leu Ser Leu
        435                 440                 445
Ser Pro Gly Lys
    450

<210>  5
<211>  1356
<212>  DNA
<213>  Artificial Sequence

<220>
<223>  Laboratory synthesis

<400>  5
gaggtgcagc tggtcgagtc cggcggagga ctggtccaac ccggtggatc tttaagactg      60
tcttgtgccg cctccggcta cgtcttcacc gactacggaa tgaactgggt gagacaagct     120
cccggtaagg gtttagagtg gatgggctgg atcaacacct atatcggcga gcccatctac     180
gctgactccg tgaagggtcg tttcaccttt tctttagata ccagcaagag caccgcctac     240
ctccagatga actctttaag ggctgaggat acagccgtgt actattgcgc cagaggctac     300
agaagctacg ccatggacta ttggggacaa ggtacactgg ttaccgtgag ctccgcctcc     360
accaaaggtc cctccgtgtt tcctctggcc cccagctcca aaagcacaag cggcggaacc     420
gccgctctgg gatgtctcgt gaaggactat tttcccgagc ccgtgaccgt tagctggaac     480
tccggagctt taacatccgg cgtgcatacc tttcccgctg tgctccagtc cagcggttta     540
tactctttat cctccgtggt gaccgtcccc tccagctctt taggcaccca gacctacatc     600
tgtaatgtga accacaagcc ttccaacacc aaggtggaca agaaagtgga gcccaaaagc     660
tgcgataaaa cccacacttg tcccccttgc cccgctcccg aactgctggg aggaggagga     720
tccggcccct ctgtgttttt attccctcct aaacccaagg acactttaat gatctctcgt     780
acacccgagg tcacatgtgt cgtcgtggac gtgtcccatg aagaccccga ggtcaagttc     840
aactggtacg tggacggagt cgaggtgcac aacgccaaaa ccaagcctcg tgaggagcag     900
tacaactcca cctatcgtgt ggtgtccgtg ctgaccgtgc tgcaccaaga ttggctgaac     960
ggcaaggagt acaagtgcaa ggtgagcaat aaggctttac ccgcccccat cgaaaagacc
1020
atctccaaag ccaagggcca gcccagagag ccccaagttt atactttacc cccctctcgt
1080
gaggagatga ccaagaatca agtttcttta acttgtctgg tcaaggcttt ctatccctcc
1140
gacatcgctg tcgagtggga gagcaacggc cagcccgaga ataactataa gaccaccccc
1200
cccgttctgg acagcgacgg ttcttttttt ttatactcca agctgaccgt ggacaagtcc
1260

agatggcagc aaggtaacgt gttcagctgc agcgtgatgc acgaggcttt acacaaccac
1320
tacacccaaa agagcctctc tttaagcccc ggtaaa
1356

<210> 6
<211> 642
<212> DNA
<213> Artificial Sequence

<220>
<223> Laboratory synthesis

<400> 6
gacatccaga tgacccagag ccctagctct ttaagcgcca gcgtgggcga tagggtgacc 60
attacttgta aggccagcca gaacgtggga accaatgtgg cttggtacca gcagaagccc 120
ggcaaggccc ccaaggcttt aatctacagc gctagctttt tatacagcgg cgtgccctac 180
agatttagcg gcagcggcag cggcaccgat tttactttaa ccatcagctc tttacagccc 240
gaggacttcg ccacctatta ctgccagcag tacaacatct accccctcac cttcggccaa 300
ggtacaaagg tggagatcaa gaggaccgtg gccgccccca gcgtgttcat cttccccccc 360
agcgacgagc agctgaagag cggcaccgcc agcgtggtgt gtttactgaa caacttctac 420
cctagggagg ccaaggtgca gtggaaggtg gacaatgctt tacagagcgg caatagccaa 480
gaaagcgtga ccgagcaaga ttccaaggac agcacctact ctttatccag cactttaaca 540
ctcagcaagg ccgactacga gaagcacaag gtgtacgctt gtgaggtgac ccaccaaggt 600
ttaagctccc ccgtgacaaa gagcttcaat aggggcgagt gc
642